(19) Europäisches Patentamt
European Patent Office
Office européen des brevets

(11) Veröffentlichungsnummer: **0 181 618 B1**

(12) **EUROPÄISCHE PATENTSCHRIFT**

(45) Veröffentlichungstag der Patentschrift: **25.03.92**

(51) Int. Cl.$^5$: **C07D 211/14**, C07D 211/12

(21) Anmeldenummer: **85114232.3**

(22) Anmeldetag: **08.11.85**

# (54) Verfahren zur Herstellung von Substituierten Piperidinen.

(30) Priorität: **16.11.84 DE 3441929**

(43) Veröffentlichungstag der Anmeldung:
**21.05.86 Patentblatt 86/21**

(45) Bekanntmachung des Hinweises auf die Patenterteilung:
**25.03.92 Patentblatt 92/13**

(84) Benannte Vertragsstaaten:
**AT BE CH DE FR GB IT LI NL SE**

(56) Entgegenhaltungen:
**GB-A- 726 378**

**BULLETIN OF THE CHEMICAL SOCIETY OF JAPAN, Band 49, Nr. 8, August 1976, Seiten 2302-2305; Y. WATANABE et al.: "The facile synthesis of N-substituted piperidines from glutaraldehyde and primary amines with tetracarbonylhydridoferrate"**

**JOURNAL OF HETEROCYCLIC CHEMISTRY, Band XII, Februar, April, Juni 1975, Seiten 161-164; A.T. NIELSEN: "Synthesis of 7-(N-alkylamino)-and 7-(N,N-dialkylamino)-1,3,5-triazaadamantanes"**

(73) Patentinhaber: **BASF Aktiengesellschaft**
**Carl-Bosch-Strasse 38**
**W-6700 Ludwigshafen(DE)**

(72) Erfinder: **Himmele, Walter, Dr.**
**Eichenweg 14**
**W-6909 Walldorf(DE)**
Erfinder: **Wiersdorff, Walter-Wielant**
**Blockteldstrasse 15**
**W-6704 Mutterstadt(DE)**
Erfinder: **Thyes, Marco, Dr.**
**Thorwaldsenstrasse 1**
**W-6700 Ludwigshafen(DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



Rank Xerox (UK) Business Services

## Beschreibung

Die vorliegende Erfindung betrifft ein Verfahren zur Herstellung von in 3- und/oder 4-Stellung substituierten Piperidinen, die gegebenenfalls noch am Stickstoff alkyliert sind.

Piperidine haben in der Wirkstoffsynthese große Bedeutung. Sowohl für Pharmawirkstoffe als auch für Wirkstoffe, die im Pflanzenschutz Einsatz finden, werden verschiedenartig substituierte Piperidine benötigt.

Ihre Herstellung erfolgt in der Regel durch Reduktion von substituierten Pyridinen, Pyridiniumsalzen oder cyclischen Amiden (Houben-Weyl, Bd. 11/1, 1957, Seiten 693 bis 695, 698, 728, 593/594). Zur Synthese aus acyclischen Edukten geht man z.B. von substituierten 1,5-Dihalogeniden, 1,5-Halogenaminen, 1,5-Aminoalkoholen, 1,5-Diaminen oder N-Arylglutarimid aus (Houben-Weyl, Bd. 11/1, 1957, Seite 117 und 255 sowie Org. Synth., Coll. Vol. IV, 1963, Seite 795 und 816).

Zum Aufbau von Piperidinen durch reduktive Aminierung von Glutardialdehyd gibt es nur wenige Beispiele. So wird in J. Heterocycl. Chem. 12 (1975) 1, 161 die Umsetzung von 7-Amino-1,3,5-triazaadamantan mit Glutardialdehyd und Wasserstoff in Gegenwart eines Platinoxid-Katalysators beschrieben. Mit Hilfe von Tetracarbonylhydridoferrat können N-substituierte Piperidine ausgehend von Glutardialdehyd und primären Aminen hergestellt werden (Y. Watanabe et al., Bull. Chem. Soc. Jp. 49 (1976) 8, 2302). Von C.F. Lane wird Natriumcyanoborhydrid als selektives Reduktionsmittel empfohlen, um z.B. 1,4- oder 1,5-Dicarbonylverbindungen mit primären Aminen oder Ammoniak zu 5- bzw. 6-gliedrigen Heterocyclen umzusetzen (Synthesis 1975, 135). C-substituierte Piperidine sind bisher noch nicht durch reduktive Aminierung eines Glutardialdehydderivates synthetisiert worden.

Trotz Variation der verschiedenen Synthesemethoden ist es schwierig, bestimmte Substitutionsmuster zu erhalten. Der Erfindung lag daher die Aufgabe zugrunde, einen einfachen Zugang zu substituierten Piperidinen zu finden, insbesondere zu solchen, die in 3- und 4-Stellung und ggf. zusatzlich noch am Stickstoff substituiert sind.

Es wurde nun gefunden, daß man in 3- und/oder 4-Stellung substituierte Piperidine, der allgemeinen Formel (I)

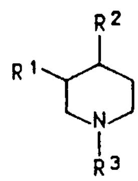

(I)

in der die Reste $R^1$ und $R^2$ gleich oder verschieden sind und einen verzweigten oder unverzweigten Alkylrest, einen Cycloalkylrest, einen Aryl- oder Aralkylrest bedeuten oder miteinander unter Ausbildung eines 5- oder 6-gliedrigen Ringes verbunden sind oder in der einer der Reste $R^1$ oder $R^2$ ein Wasserstoffatom sein kann und in der $R^3$ für einen $C_1$- bis $C_4$-Alkylrest oder für Wasserstoff steht, besonders vorteilhaft dadurch herstellen kann, daß man in 2- und/oder 3-Stellung substituierte Glutardialdehyde der allgemeinen Formel (II)

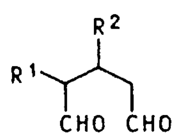

(II)

in der die Reste $R^1$ und $R^2$ die obengenannte Bedeutung haben, mit Ammoniak oder einem primären Amin mit 1 bis 4 Kohlenstoffatomen und Wasserstoff bei erhöhter Temperatur und erhöhtem Druck in Gegenwart eines Hydrierkatalysators zu dem Piperidinderivat (I) umsetzt.

Die aminierende Hydrierung von in 2- und/oder 3-Stellung substituierten Glutardialdehyden, die nach folgendem Reaktionsschema verläuft, liefert in guter Ausbeute und Selektivität in 3- und/oder 4-Stellung substituierte Piperidine

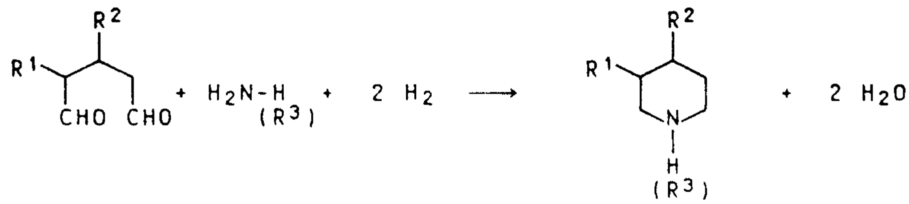

(II) (I)

Die Glutardialdehyde (II) werden zweckmäßigerweise durch saure Hydrolyse von in 4- und/oder 5-Stellung substituierten 2-Alkoxy-3,4-dihydropyranen, z.B. wie in der DE-OS 968 511 beschrieben, hergestellt. Da sie in reiner Form nicht sehr stabil sind, werden sie in Form von Losungsmittel-Glutardialdehyd-Gemischen verwendet. Die Lösungsmittelkomponente, die dem Dial (II) zur Stabilisierung zugesetzt wird, sollte in ihrem Löslichkeitsverhalten so beschaffen sein, daß es während der Umsetzung nicht zur Ausbildung einer zweiten flüssigen Phase kommt. Gut geeignet sowohl fur den Zweck der Stabilisierung des Dialdehyds als auch für die aminierende Hydrierung des Dial-Lösungsmittelgemisches sind Dioxan oder niedermolekulare Dialkylether eines Glykols, z.B. Ethylenglykol-dimethylether oder Ethylenglykol-diethylether. Besonders geeignet ist Tetrahydrofuran.

In vielen Fällen ist es möglich, die nach der Spaltung der 6-Alkoxy-dihydropyrane erhaltenen rohen Reaktionsgemische nach Erhitzen und Abdestillieren der freien Ameisensäure direkt zur aminierenden Hydrierung weiterzuverwenden, so daß auf diese Weise ein Arbeitsgang, nämlich die Reinigung des Dials (II) durch Destillation, gespart wird, wodurch Substanzverluste vermieden werden.

Für das erfindungsgemäße Verfahren in Betracht kommende Glutardialdehyde (II) sind solche, in denen die Reste $R^1$ und/oder $R^2$ verzweigte oder unverzweigte Alkylgruppen, z.B. mit je 1 bis 20, insbesondere 1 bis 10, vorteilhaft 1 bis 5 Kohlenstoffatomen bedeuten, also z.B. Methyl-, Ethyl-, Propyl-, Isopropyl-, Butyl-, Isobutyl-, tert.-Butyl-, Pentyl- oder Isopentylgruppen. Die Alkylreste können noch durch unter den Reaktionsbedingungen indifferente Gruppen, z.B. Alkoxygruppen mit 1 bis 4 Kohlenstoffatomen substituiert sein.

Weiterhin kommen für $R^1$ und/oder $R^2$ Cycloalkylreste mit 5 bis 8 Kohlenstoffatomen in Frage. Beide Reste $R^1$ und $R^2$ können auch miteinander unter Ausbildung eines 5- oder 6-gliedrigen Ringes verbunden sein.

Die Reste $R^1$ und/oder $R^2$ können auch Arylgruppen mit 6 bis 15, oder Aralkylgruppen mit 7 bis 16 Kohlenstoffatomen bedeuten. Neben Alkylsubstituenten wie Methyl-, Ethyl-, Propyl-, Isopropyl-, Butyl-, Isobutyl-, tert.-Butyl-, Pentyl- und Isopentyl können die aromatischen Kerne noch durch unter den Reaktionsbedingungen inerte Gruppen wie die Alkoxygruppe oder Halogen substituiert sein.

Die beiden Reste $R^1$ und $R^2$ können gleich oder verschieden sein und einer der beiden Reste $R^1$ oder $R^2$ kann auch für Wasserstoff stehen.

Als Aminkomponente kommen niedermolekulare Alkylamine mit 1 bis 4 Kohlenstoffatomen in Betracht, z.B. Butyl-, Propyl- und Ethylamin. Besonders geeignet sind bei Raumtemperatur gasförmige Amine, z.B. Methylamin und vor allem Ammoniak. Die Aminkomponente wird zweckmäßig im Überschuß zu (II) eingesetzt. Dabei werden in der Regel 1,1 bis 7,0, insbesondere 1,5 bis 4 mol Amin bzw. Ammoniak pro Mol Glutardialdehyd (II) verwendet.

Um bei der aminierenden Hydrierung eine Ausbildung von zwei flüssigen Phasen im Autoklaven zu vermeiden, ist es zweckmäßig, die Menge an Amin bzw. Ammoniak mit einem niedermolekularen Alkohol, z.B. Methanol, Ethanol oder iso-Propanol zu vermischen. Höhere Alkohole, die bereits mit wasserfreiem Ammoniak zweiphasige Gemische bilden, sind nicht gut geeignet.

Die aminierende Hydrierung von (II) wird z.B. bei Temperaturen oberhalb von 50 °C und unterhalb von 200 °C, insbesondere bei 70 bis 120 °C, vorteilhaft 80 bis 110 °C in Gegenwart eines geeigneten Hydrierkatalysators nach den dafür üblichen Techniken unter Druck durchgeführt.

Zur Herstellung der Hydrierkatalysatoren finden z.B. die Platinmetalle Ruthenium, Rhodium, Palladium, Iridium und Platin sowie Nickel und Kobalt Verwendung. Die Katalysatoren können trägerlos, wie beispielsweise Raney-Nickel, Raney-Kobalt, oder als Trägerkatalysatoren eingesetzt werden. Die trägerlosen Katalysatoren können auch Verbindungen der erfindungsgemäßen Metalle, bevorzugt ihre Oxide sein. Als Träger eignen sich beispielsweise Kohle, Kieselgel, Aluminiumsilikat oder Aluminiumoxid. Die Herstellung solcher Trägerkatalysatoren kann in beliebiger Weise, z.B. durch Tränken des Trägers mit entsprechenden Lösungen der Metallsalze, durch Verkneten bzw. Mischen unter Vermahlen der Komponenten, erfolgen. Bezüglich Details der Herstellung von Katalysatoren, insbesondere Trägerkatalysatoren, wird auf Houben-Weyl, Methoden der Organischen Chemie, Band 4/2, Seiten 137 ff. verwiesen. Bei Trägerkatalysatoren

beträgt der Metallgehalt des Katalysators, bezogen auf die Gewichtsmenge Trägermaterial, gewöhnlich von 0,05 bis 19,5, vorzugsweise 0,5 bis 15 Gew.%. Das Metall des Hydrierkatalysators wird in der Regel in Mengen von 0,1 bis 100, insbesondere 0,5 bis 20 Gew.%, bezogen auf Ausgangsstoff II, verwendet.

Als Hydrierkatalysatoren haben sich insbesondere Raney-Kobalt und Raney-Nickel bewährt. Nach erfolgter Umsetzung kann der Katalysator durch Filtration oder Sedimentation vom Reaktionsgemisch abgetrennt und vorteilhaft für eine weitere Hydrierung verwendet werden.

Der Wasserstoffdruck kann in weiten Grenzen variiert werden. Der zu wählende Druck hängt zur Erzielung optimaler Ausbeute von der Intensität der Rührung des Reaktionsgemisches ab. Da die Reaktion in der flüssigen Phase verläuft, muß die Bereitstellung des Wasserstoffes für die Hydrierreaktion am suspendierten Katalysator sowohl durch die Löslichkeit als auch durch die Nachlieferung für die Umsetzung optimal abgestimmt sein. Mit niederen Wasserstoffpartialdrucken kann bei intensiverer Rührung gearbeitet werden, dagegen erweist es sich als vorteilhaft die Reaktion bei höherem Wasserstoffpartialdruck ablaufen zu lassen, wenn der Autoklav mit einem weniger intensiv ruhrenden Ankerruhrer gemischt wird.

Für das erfindungsgemäße Verfahren sind insbesondere Magnethubrührautoklaven gut geeignet, in denen nach Vorlegen des Katalysators in Alkohol, Einpressen der Aminkomponente und Erwärmen auf die Reaktionstemperatur durch Nachpressen von Wasserstoff ein Gesamtdruck von 100 bis 300, insbesondere 100 bis 200, vorzugsweise 130 bis 170 bar eingestellt wird.

Man arbeitet vorteilhafterweise so, daß man vor der Zugabe des "Glutardialdeyhd-Lösungsmittelgemisches" die Reaktionsbedingungen wie Druck und Temperatur einstellt und dann die Zugabe, die vorteilhaft unter guter Begasung und Rühren erfolgt, gleichmäßig über einen Zeitraum von mehreren Stunden, in der Regel 5 bis 10, insbesondere 6 bis 8 Stunden verteilt. Nachdem der stabilisierte Glutardialdehyd in den Autoklaven eingepreßt worden ist, wird das Reaktionsgemisch zweckmäßig noch mehrere Stunden, in der Regel 2 bis 6 Stunden unter den Reaktionsbedingungen weitergerührt.

Die Aufarbeitung kann vorteilhaft in der Weise erfolgen, daß man z.B. das Reaktionsgemisch abkühlt, wobei der überschüssige Ammoniak bzw. das Amin und der Wasserstoff während der Abkühlungsphase ausgegast werden, um so die Abkühlung zu beschleunigen. Nach Abtrennen des Katalysators wird der Alkohol und das Dial-Lösungsmittel sowie das bei der Reaktion erzeugte Wasser abdestilliert. Sowohl das Lösungsmittel als auch die Aminkomponente können für weitere Umsetzungen wiederverwendet werden.

Die nach dem erfindungsgemäßen Verfahren hergestellten Piperidine können zweckmäßig durch fraktionierte Destillation aus dem verbleibenden Reaktionsgemisch gewonnen werden. Die Destillation wird nach den dafür üblichen Techniken, bei Normaldruck oder insbesondere bei größeren Substituenten unter vermindertem Druck durchgeführt.

Durch die erfindungsgemäße aminierende Hydrierung von (II) werden die substituierten Piperidine in guter Ausbeute und hoher Reinheit erhalten. Bei der Synthese der in 3- und 4-Stellung substituierten Produkte überwiegt die trans-Konfiguration der Reste $R^1$ und $R^2$.

Die nachfolgenden Beispiele sollen die Erfindung näher erläutern. Im Beispiel 12 wird gezeigt, wie sich durch Alkylierung am Stickstoff aus den in 3- und/oder 4-Stellung substituierten Piperidinen Verbindungen mit fungizider Wirksamkeit herstellen lassen. Auch zum Aufbau von Verbindungen mit wertvollen pharmakologischen, z.B. thrombozytenaggregationshemmenden Eigenschaften, dienen substituierte, insbesondere arylsubstituierte Piperidine als Bausteine. So sind nach dem in der DE-OS 33 02 021 auf Seite 7 bis 9 beschriebenen Verfahren z.B. 6-Aryl-4,5-dihydro-3(2H)-pyridazinone der Formel

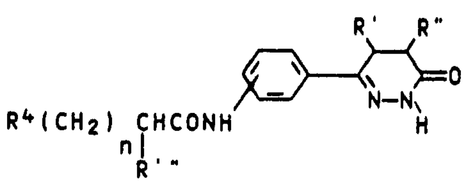

zugänglich, in denen $R^4$ einen in 3- und/oder 4-Stellung substituierten Piperidylrest bedeutet, indem man die entsprechende Halogenverbindung mit am Stickstoff unsubstituierten Piperidinen gegebenenfalls in Gegenwart einer Hilfsbase und/oder eines Iodids als Katalysator umsetzt.

Beispiel 1

Synthese des 4-Phenyl-piperidins durch aminierende Hydrierung von destilliertem 3-Phenylglutardial

In einem 5-1-Magnethubrührautoklaven werden 100 g Raney-Kobalt in 625 g Methanol eingefüllt. Der

Autoklav wird druckdicht verschlossen und mit Stickstoff gespült. Nachdem 500 g Ammoniak eingepreßt worden sind, wird der Autoklav auf 110 °C aufgeheizt und durch Nachpressen von Wasserstoff ein Gesamtdruck von 150 bar eingestellt. Die Rührung wird eingeschaltet. In den Autoklaven wird im Verlauf von 7 Stunden jeweils 200 g/h ein Gemisch aus 938 g destilliertem ca. 99 %igem 3-Phenylglutardialdehyd und 469 g Tetrahydrofuran zugepumpt. Zum Freispülen der Einpreßleitung werden nach Zupumpen des Dialgemisches 200 g Tetrahydrofuran nachgepumpt. Nach Einpressen dieser Glutardialdehydlösung wird der Autoklav noch weitere 6 Stunden unter den genannten Reaktionsbedingungen gehalten. Verbrauchter Wasserstoffdruck wird stündlich nachgepreßt, um den erwünschten Wasserstoffpartialdruck aufrechtzuhalten.

Nach Ende der Reaktionszeit wird auf 60 °C abgekühlt und mit der Entspannung des Wasserstoffs und eines Teils des überschüssigen Ammoniaks begonnen.

Nach dem Entspannen des noch im Autoklaven vorhandenen Gases, wird mit Stickstoff gespült, der Autoklaveninhalt entnommen und durch Filtration vom suspendierten Raney-Kobalt abgetrennt.

In einer Kurzweg-Destillationsapparatur werden bei 67 mbar zunächst Ammoniak, Tetrahydrofuran und Methanol abgezogen. Wenn diese Komponenten abdestilliert sind, wird durch Heizen und Druckabsenkung auf 40 mbar das bei der Reaktion entstandene Wasser übergetrieben. Bei dem gleichen Druck geht das gebildete 4-Phenylpiperidin in einem Temperaturbereich zwischen 146 und 156 °C über. Es werden 654 g 4-Phenylpiperidin gewonnen. Nach der gaschromatographischen Analyse besteht diese Fraktion aus über 99,5 %igem 4-Phenylpiperidin. Das Amin erstarrt kristallin in langen Nadeln. Der Schmelzpunkt liegt bei 63 °C. Die Ausbeute bezogen auf das eingesetzte 3-Phenyl-glutardial liegt bei 59,6 %. Die anfallende Qualität entspricht den Anforderungen zur Weiterverarbeitung für Wirkstoffsynthesen.

Beispiel 2

Synthese des 4-Pheny-piperidins durch aminierende Hydrierung von nicht destilliertem 3-Phenylglutardial

1165 g 3-Phenyl-glutardial, das bei einer mit Ameisensäure bewirkten Acidolyse von 4-Phenyl-6-isobutoxy-5,6-dihydropyran (1560 g) erhalten wurde, wurde durch Andestillieren von Lösungsmittel und Ameisensäure befreit, zur Stabilisierung mit 291 g Tetrahydrofuran vermischt und wie unter Beispiel 1 erläutert zum 4-Phenyl-piperidin umgesetzt.

Bei der Aufarbeitung des Reaktionsgemisches (2627 g) durch Filtration, Lösungsmittelabtrennung und anschließende Fraktionierung des 4-Phenyl-piperidins werden bei einem Druck von 40 mbar und Übergangstemperaturen von 146 bis 164 °C 736 g reines über 99,5 %iges 4-Phenyl-piperidin erhalten. In einem bei der Destillation erhaltenen Nachlauf von 81 g sind noch weitere 57 g Amin vorhanden. Dieser Nachlauf enthält jedoch nur etwa 70 % 4-Phenyl-piperidin.

An direkt für Wirkstoffsynthesen verwertbarem Amin beträgt die Ausbeute 68,1 %, bezogen auf das in die Reaktion eingebrachte 4-Phenyl-6-isobutoxy-5,6-dihydropyran. Wird die im Nachlauf vorhandene Menge an Amin berücksichtigt, dann errechnet sich eine Ausbeute von 73,2 %.

Die Beispiele 3 bis 11, die analog Beispiel 1 durchgeführt worden sind, sind in Tabelle 1 zusammengestellt:

Tabelle 1

Synthese von in 3- und 4-Stellung substituierten Piperidinen durch aminierende Hydrierung von substituierten Glutardialdehyden

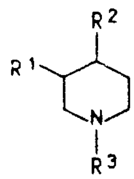

| Beispiel Nummer | $R^1$ | $R^2$ | $R^3$ | Kp: $^{\circ}$C/mbar | Ausbeute an subst. Piperidin in % | cis-trans-Verhältnis der Substituenten $R^1$ und $R^2$ cis:trans in % |
|---|---|---|---|---|---|---|
| 3 | $CH_3$ | $C_2H_5$ | H | 106/200 | 82 | |
| 4 | $C_2H_5$ | $n-C_3H_7$ | H | 99/ 32 | 88 | |
| 5 | $n-C_3H_7$ | $n-C_4H_9$ | H | 123/ 27 | 91 | |
| 6 | $i-C_3H_7$ | $i-C_4H_9$ | H | 125/ 37 | 87 | |
| 7 | $-CH_3$ | 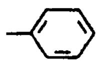 | H | 140/ 20 | 73 | 15:85 |
| 8 | $CH_3$ | 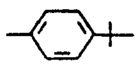 | H | 156/2,7 | 75 | 17:83 |
| 9 | $C_2H_5$ | $n-C_3H_7$ | $-CH_3$ | 129/146 | 58 | |
| 10 | H | 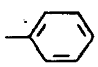 | $-CH_3$ | 130/ 29 | 72 | |
| 11 | $CH_3$ | 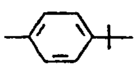 | $-CH_3$ | 118/0,3 | 60 | 35:65 |

Beispiel 12

Alkylierung des 4-(4'-tert.-Butyl-phenyl)-3-methylpiperidins. Synthese des N-Hexahydrobenzyl-4-(4'-tert.-butyl-phenyl)-3-methyl-piperidins (X)

30 g 4-(4'-tert.-Butyl-phenyl)-3-methyl-piperidin (aus Beispiel 8) werden mit 17,5 g Hexahydrobenzaldehyd vermischt. Nach zweistündigem Stehen werden 36 g Ameisensäure zugegeben. Dieses Reaktionsgemisch wird 12 Stunden am Rückfluß erhitzt.

Durch Fraktionierung in einer Kurzwegdestillation werden die folgenden Fraktionen gewonnen:

| | |
|---|---|
| 1. bis 185 ° C/7 mbar | 4 g Nach GC-Analyse |
| 2. bis 190 ° C/7 mbar | 31 g ca. 86 % (X) |
| Destillationsrückstand | 4 g |

Die NMR- und IR-Spektren bestatigen das Vorliegen der Verbindung (X), die gemäß der deutschen Anmeldung Nr. P 31 44 927.6, als Fungizid verwendbar ist. Als Verunreinigung liegt noch das N-Formylprodukt des Eduktes vor.

**Patentansprüche**

**1.** Verfahren zur Herstellung von in 3- und 4-Stellung substituierten Piperidinen, der allgemeinen Formel (I),

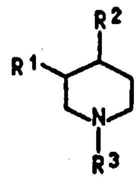

(I)

in der die Reste $R^1$ und $R^2$ gleich oder verschieden sind und einen verzweigten oder unverzweigten Alkylrest, einen Cycloalkylrest, einen Aryl-oder Aralkylrest bedeuten oder miteinander unter Ausbildung eines 5- oder 6-gliedrigen Ringes verbunden sind oder in der einer der Reste $R^1$ oder $R^2$ ein Wasserstoffatom sein kann und in der $R^3$ für einen $C_1$- bis $C_4$-Alkylrest oder für Wasserstoff steht, dadurch gekennzeichnet, daß man in 2- und/oder 3-Stellung substituierte Glutardialdehyde der allgemeinen Formel (II),

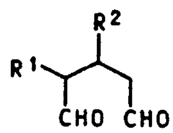

(II)

in der die Reste $R^1$ und $R^2$ die obengenannte Bedeutung haben, mit Ammoniak oder einem primären Amin mit 1 bis 4 Kohlenstoffatomen und Wasserstoff bei erhöhter Temperatur und erhöhtem Druck in Gegenwart eines Hydrierkatalysators zu dem Piperidinderivat (I) umsetzt.

**2.** Verfahren nach Anspruch 1, dadurch gekennzeichnet, daß man die Umsetzung bei Temperaturen von 70 bis 140 ° C, durchführt.

**3.** Verfahren nach Anspruch 1, dadurch gekennzeichnet, daß man als Hydrierkatalysator Raney-Kobalt oder Raney-Nickel verwendet.

**4.** Verfahren nach Anspruch 1, dadurch gekennzeichnet, daß man als Aminkomponente Ammoniak oder Methylamin verwendet.

**5.** Verfahren nach Anspruch 4, dadurch gekennzeichnet, daß man 1,1 bis 7,0 mol der Aminkomponente pro Mol substituierten Glutardialdehyd (II) verwendet.

**6.** Verfahren nach Anspruch 1, dadurch gekennzeichnet, daß man die Aminkomponente vor der Umsetzung mit (II) in einem niedermolekularen Alkohol löst.

**7.** Verfahren nach Anspruch 6, dadurch gekennzeichnet, daß man als niedermolekularen Alkohol Methanol. Ethanol oder iso-Propanol verwendet.

**8.** Verfahren nach Anspruch 1, dadurch gekennzeichnet, daß man die Umsetzung bei einem Gesamtdruck von 50 bis 300 bar durchführt.

**9.** Verfahren nach Anspruch 1, dadurch gekennzeichnet, daß man den substituierten Glutardialdehyd (II) im Gemisch mit einem inerten Lösungsmittel verwendet.

**10.** Verfahren nach Anspruch 9, dadurch gekennzeichnet, daß man als Verdünnungsmittel für den Glutardialdehyd Tetrahydrofuran, Dioxan oder einen niedermolekularen Dialkylether eines Glykols verwendet.

**11.** Verfahren nach Anspruch 1, dadurch gekennzeichnet, daß man den Katalysator nach der Umsetzung abtrennt und das Piperidinderivat (I) durch fraktionierte Destillation aus dem rohen Reaktionsgemisch gewinnt.

**Claims**

**1.** A process for the preparation of a piperidine which is substituted in the 3- and 4-position and is of the formula (I)

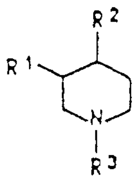

( I )

where $R^1$ and $R^2$ are identical or different and are each straight-chain or branched alkyl, cycloalkyl, aryl or aralkyl, or may be bonded to one another to form a 5-membered or 6-membered ring, or one of the radicals $R^1$ or $R^2$ may be hydrogen, and $R^3$ is $C_1$-$C_4$-alkyl or hydrogen, wherein a glutardialdehyde which is substituted in the 2-and/or 3-position and is of the formula (II)

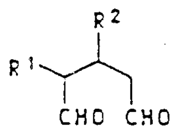

( II )

where $R^1$ and $R^2$ have the above meanings, is reacted with ammonia or a primary amine of 1 to 4 carbon atoms and hydrogen at elevated temperatures and under superatmospheric pressure in the presence of a hydrogenation catalyst to give a piperidine derivative (I).

**2.** A process as claimed in claim 1, wherein the reaction is carried out at from 70 to 140 ° C, in particular from 80 to 110 ° C.

**3.** A process as claimed in claim 1, wherein Raney cobalt or Raney nickel is used as the hydrogenation catalyst.

**4.** A process as claimed in claim 1, wherein ammonia or methylamine is used as the amine component.

**5.** A process as claimed in claim 4, wherein from 1.1 to 7.0 moles of the amine component are used per mole of substituted glutardialdehyde (II).

**6.** A process as claimed in claim 1, wherein the amine component is dissolved in a low molecular weight alcohol before being reacted with (II).

**7.** A process as claimed in claim 6, wherein methanol, ethanol or isopropanol is used as the low molecular weight alcohol.

**8.** A process as claimed in claim 1, wherein the reaction is carried out under a total pressure of from 50 to 300 bar.

**9.** A process as claimed in claim 1, wherein the substituted glutardialdehyde (II) is used as a mixture with an inert solvent.

**10.** A process as claimed in claim 9, wherein tetrahydrofuran, dioxane or a low molecular weight dialkyl ether of a glycol is used as a diluent for the glutardialdehyde.

**11.** A process as claimed in claim 1, wherein the catalyst is separated off after the reaction, and the piperidine derivative (I) is isolated from the crude reaction mixture by fractional distillation.

**Revendications**

**1.** Procédé de préparation de pipéridines substituées en position 3 et 4 de formule générale I

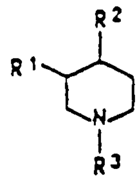

(I)

dans laquelle les restes $R^1$ et $R^2$ sont identiques ou différents et représentent un reste alkyle ramifié ou non ramifié, un reste cycloalkyle, un reste aryle ou aralkyle ou sont liés l'un à l'autre avec formation d'un noyau à 5 ou 6 chaînons ou dans laquelle l'un des restes $R^1$ et $R^2$ peut être un atome d'hydrogène et dans laquelle $R^3$ est mis pour un reste alkyle en $C_1$-$C_4$ ou pour un atome d'hydrogène, caractérisé en ce qu'on transforme en le dérivé de pipéridine (I) des glutardialdéhydes substitués en position 2 et/ou 3 de formule générale (II)

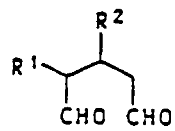

(II)

dans laquelle les restes $R^1$ et $R^2$ ont les significations données précédemment, avec de l'ammoniac ou une amine primaire ayant de 1 à 4 atomes de carbone et de l'hydrogène, à température élevée et pression élevée, en présence d'un catalyseur d'hydrogénation.

**2.** Procédé selon la revendication 1, caractérisé en ce qu'on effectue la transformation à des températures de 70 à 140 ° C, en particulier de 80 à 110 ° C.

**3.** Procédé selon la revendication 1, caractérisé en ce qu'on utilise comme catalyseur d'hydrogénation du cobalt de Raney ou du nickel de Raney.

**4.** Procédé selon la revendication 1, caractérisé en ce qu'on utilise comme composant amine de l'ammoniac ou de la méthylamine.

**5.** Procédé selon la revendication 4, caractérisé en ce qu'on utilise de 1,1 à 7,0 moles de composant amine par mole de glutardialdéhyde (II) substitué.

**6.** Procédé selon la revendication 1, caractérisé en ce qu'on dissout le composant amine dans un alcool de faible poids moléculaire avant la réaction avec le composé (II).

**7.** Procédé selon la revendication 6, caractérisé en ce qu'on utilise comme alcool de faible poids moléculaire du méthanol, de l'éthanol ou de l'isopropanol.

**8.** Procédé selon la revendication 1, caractérisé en ce qu'on effectue la transformation à une pression totale de 50 à 300 bar.

**9.** Procédé selon la revendication 1, caractérisé en ce qu'on utilise le glutardialdéhyde substitué (II) en melange avec un solvant inerte.

**10.** Procédé selon la revendication 9, caractérisé en ce qu'on utilise comme milieu de dilution du glutardialdéhyde du tétrahydrofuranne, du dioxanne ou un éther dialkylique de faible poids moléculaire d'un glycol.

**11.** Procédé selon la revendication 1, caractérisé en ce qu'on sépare le catalyseur après la transformation

et on récupère le dérivé de pipéridine (I) par distillation fractionnée à partir du mélange réactionnel brut.